(19) Europäisches Patentamt
European Patent Office
Office européen des brevets

(11) **EP 2 270 203 A1**

(12) **EUROPEAN PATENT APPLICATION**

(43) Date of publication:
**05.01.2011 Bulletin 2011/01**

(51) Int Cl.:
***C12Q 1/68*** *(2006.01)*

(21) Application number: **09164052.4**

(22) Date of filing: **29.06.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(71) Applicant: **AIT Austrian Institute of Technology GmbH**
**1220 Wien (AT)**

(72) Inventors:
- **Krainer, Siegfried**
**8832 Oberwölz (AT)**
- **Fluch, Silvia**
**2540 Bad Vöslau (AT)**
- **Levente, Bodrossy**
**2440 Reisenberg (AT)**
- **Stierschneider, Michael**
**1070 Vienna (AT)**

(74) Representative: **Sonn & Partner Patentanwälte**
**Riemergasse 14**
**1010 Wien (AT)**

(54) **Oligonucleotide hybridization method**

(57) The present invention provides a method to bind oligonucleotide molecules of a sample to an oligonucleotide probe with increased specificity comprising the steps of

- providing a solid support with at least one immobilized oligonucleotide DNA probe comprising an oligonucleotide probe sequence,
- in any order, contacting said probes with a sample potentially comprising oligonucleotide molecules binding said probes and contacting said probes with at least one competitive non-DNA oligonucleotide, wherein said competitive oligonucleotide comprises a sequence which is complementary to the oligonucleotide probe sequence; and/or as an alternative or in addition, in any order, contacting said probes with a sample comprising oligonucleotide molecules and contacting said oligonucleotide molecules with at least one competitive non-DNA oligonucleotide, wherein said competitive oligonucleotide comprises a sequence of the oligonucleotide probe sequence,
- incubating said contacted probes with the oligonucleotide molecules of the sample and competitive oligonucleotides at a temperature of between 15°C below the melting temperature of DNA duplex oligonucleotides of the probe sequence up to said melting temperature,

thereby increasing binding specificity of the oligonucleotide probe to an oligonucleotide molecule comprising a sequence complementary to said probe sequence; as well as means for performing said method.

EP 2 270 203 A1

SEQUENCE LISTING

<110>  AIT Austrian Institute of Technology GmbH

<120>  Oligonucleotide Hybridization Method

<130>  R 54593

<160>  59

<170>  PatentIn version 3.5

<210>  1
<211>  20
<212>  DNA
<213>  Artificial Sequence

<220>
<223>  primer

<400>  1
actgggatga yatggagaag                                                    20

<210>  2
<211>  20
<212>  DNA
<213>  Artificial Sequence

<220>
<223>  primer

<400>  2
aycctccaat ccagacactg                                                    20

<210>  3
<211>  18
<212>  DNA
<213>  Artificial Sequence

<220>
<223>  primer

<400>  3
taatacgact cactatag                                                      18

<210>  4
<211>  60
<212>  DNA
<213>  Artificial Sequence

<220>
<223>  artificial oligonucleotide probe

<400>  4
actttctaca atgagcttcg tgttgccccc gaggagcacc ctgttctgct cactgaagca        60

<210>  5
<211>  60
<212>  DNA
<213>  Artificial Sequence

<220>
<223>  artificial oligonucleotide probe

<400>  5
tttgccacat gccattcttc gtttggatct tgctggccgt gatttaactg ataacctgat        60

<210> 6
<211> 60
<212> DNA
<213> Artificial Sequence

<220>
<223> artificial oligonucleotide probe

<400> 6
aggtatccac gagactacct acaactctat tatgaagtgt gatgttgata tcaggaagga 60

<210> 7
<211> 60
<212> DNA
<213> Artificial Sequence

<220>
<223> artificial oligonucleotide probe

<400> 7
aaatgaccca gattatgttt gagaccttca atgttccggc catgtatgtt gctattcagg 60

<210> 8
<211> 60
<212> DNA
<213> Artificial Sequence

<220>
<223> artificial oligonucleotide probe

<400> 8
aaattactgc tttggctccc agcagcatga agattaaggt tgttgcacca ccagagagaa 60

<210> 9
<211> 60
<212> DNA
<213> Artificial Sequence

<220>
<223> artificial oligonucleotide probe

<400> 9
aaaagctgtc atacattgcc cttgactatg agcaagagct ggagacagcg aggaccagct 60

<210> 10
<211> 60
<212> DNA
<213> Artificial Sequence

<220>
<223> artificial oligonucleotide probe

<400> 10
aaagacttgt atggtaacat tgtcctttca ggaggaacaa ccatgttccc aggaattgct 60

<210> 11
<211> 60
<212> DNA
<213> Artificial Sequence

<220>
<223> artificial oligonucleotide probe

<400> 11
aaagaaattt ctgctttggc cccaagcagc atgaagatca aggtcgttgc accacctgag 60

<210> 12
<211> 60
<212> DNA
<213> Artificial Sequence

<220>
<223> artificial oligonucleotide probe

<400> 12
aaaggccaac agagagaaaa tgacccagat tatgtttgag acattcaacg ttccggctat 60

<210> 13
<211> 60
<212> DNA
<213> Artificial Sequence

<220>
<223> artificial oligonucleotide probe

<400> 13
aaattgtccg tgacatgaag gagaagcttg cttatgtggc tcttgactac gagcaggagc 60

<210> 14
<211> 60
<212> DNA
<213> Artificial Sequence

<220>
<223> artificial oligonucleotide probe

<400> 14
acatgaagga gaagcttgct tatgtggctc ttgactacga gcaggagctt gacactgcca 60

<210> 15
<211> 60
<212> DNA
<213> Artificial Sequence

<220>
<223> artificial oligonucleotide probe

<400> 15
aagtcctctt ccagccatcc atgatcggaa tggaagctgc aggtatccat gagactacct 60

<210> 16
<211> 60
<212> DNA
<213> Artificial Sequence

<220>
<223> artificial oligonucleotide probe

<400> 16
aactggtatt gtgctggact ctggtgacgg tgtgagtcgc actgtgccta tctacgaagg 60

<210> 17
<211> 60
<212> DNA
<213> Artificial Sequence

<220>

<223> artificial oligonucleotide probe

<400> 17
aagattctca ccgagagagg gtacatgttc acaaccactg ctgagcggga aattgtccgt 60

<210> 18
<211> 60
<212> DNA
<213> Artificial Sequence

<220>
<223> artificial oligonucleotide probe

<400> 18
aaccatgttc cctggtattg ctgaccggat gagcaaggag atcactgcac ttgccccaag 60

<210> 19
<211> 60
<212> DNA
<213> Artificial Sequence

<220>
<223> artificial oligonucleotide probe

<400> 19
aacaggtatt gtcttggatt ctggtgatgg tgtgagtcat accgtgccaa tctatgaggg 60

<210> 20
<211> 60
<212> DNA
<213> Artificial Sequence

<220>
<223> artificial oligonucleotide probe

<400> 20
aactgaatct ttgatgaaga tcctcactga gagagggtat atgttcacca cctcggctga 60

<210> 21
<211> 60
<212> DNA
<213> Artificial Sequence

<220>
<223> artificial oligonucleotide probe

<400> 21
aaagagaagc ttgcctatgt tgctgtggat tatgaacaag agcttgaaac tgcaaagagc 60

<210> 22
<211> 60
<212> DNA
<213> Artificial Sequence

<220>
<223> artificial oligonucleotide probe

<400> 22
actgggatga catggagaag atttggcatc acactttcta caatgagctt cgtgttgctc 60

<210> 23
<211> 60
<212> DNA
<213> Artificial Sequence

<220>
<223> artificial oligonucleotide probe

<400> 23
atgacatgga gaagatttgg catcacactt tctacaatga gcttcgtgtt gctcctgaag 60

<210> 24
<211> 60
<212> DNA
<213> Artificial Sequence

<220>
<223> artificial oligonucleotide probe

<400> 24
acactttcta caatgagctt cgtgttgctc ctgaagagca cccagtcctc ctgactgagg 60

<210> 25
<211> 60
<212> DNA
<213> Artificial Sequence

<220>
<223> artificial oligonucleotide probe

<400> 25
ctgaggctcc tctcaaccct aaggctaaca gagagaagat gactcaaatt atgtttgaga 60

<210> 26
<211> 60
<212> DNA
<213> Artificial Sequence

<220>
<223> artificial oligonucleotide probe

<400> 26
cagaagtccc cttccagcct tctctcattg gaatggaagc tgctggcatc cacgagacta 60

<210> 27
<211> 60
<212> DNA
<213> Artificial Sequence

<220>
<223> artificial oligonucleotide probe

<400> 27
aaaaacttgc atacatggct cttgactacg aacaagagct ggagactgca aagagcagct 60

<210> 28
<211> 60
<212> DNA
<213> Artificial Sequence

<220>
<223> artificial oligonucleotide probe

<400> 28
aaaagaacta cgagcttcct gatggtcagg tcattaccat tggagctgag agattcagat 60

<210> 29
<211> 60

<212> DNA
<213> Artificial Sequence

<220>
<223> artificial oligonucleotide probe

<400> 29
aaattactgc tcttgctccc agcagcatga agatcaaggt tgtggcacca ccggagagaa 60

<210> 30
<211> 60
<212> DNA
<213> Artificial Sequence

<220>
<223> artificial oligonucleotide probe

<400> 30
aaacagggag aagatgaccc agattatgtt cgagacattc aactgcccag caatgtatgt 60

<210> 31
<211> 60
<212> DNA
<213> Artificial Sequence

<220>
<223> artificial oligonucleotide probe

<400> 31
aaaaacttgc ctacgttgcc cttgattatg aacaggagct ggagactgcc aggaccagct 60

<210> 32
<211> 60
<212> DNA
<213> Artificial Sequence

<220>
<223> artificial oligonucleotide probe

<400> 32
aaaggtttag gtgccctgag gttctattcc agccatcctt cattggcatg gaatctgctg 60

<210> 33
<211> 60
<212> DNA
<213> Artificial Sequence

<220>
<223> artificial oligonucleotide probe

<400> 33
aagatctggc atcacacatt ttataatgaa cttcgtgttg ctcccgagga gcacccagtg 60

<210> 34
<211> 60
<212> DNA
<213> Artificial Sequence

<220>
<223> artificial oligonucleotide probe

<400> 34
acttgcatat gttgccctag attatgagca agaactcgag actgcaaaaa gcagttcatc 60

<210> 35
<211> 60
<212> DNA
<213> Artificial Sequence

<220>
<223> artificial oligonucleotide probe

<400> 35
aactcgagac tgcaaaaagc agttcatcag ttgagaaaag ctatgagctt cctgatggac 60

<210> 36
<211> 60
<212> DNA
<213> Artificial Sequence

<220>
<223> artificial oligonucleotide probe

<400> 36
acagggaaaa gatgactcag atcatgtttg agacgttcaa tgtccctgcc atgtatgtcg 60

<210> 37
<211> 60
<212> DNA
<213> Artificial Sequence

<220>
<223> artificial oligonucleotide probe

<400> 37
aaaccgctaa gagtagctct tccattgaga agaactatga acttccggat gggcaagtta 60

<210> 38
<211> 60
<212> DNA
<213> Artificial Sequence

<220>
<223> artificial oligonucleotide probe

<400> 38
aaaggacttg tacggcaaca ttgtgctcag tggtggctct actatgttcc ctggtatagc 60

<210> 39
<211> 60
<212> DNA
<213> Artificial Sequence

<220>
<223> artificial oligonucleotide probe

<400> 39
ccaggccgtt ctctctctgt atgccagtgg tcgtactact ggtattgtgc ttgactctgg 60

<210> 40
<211> 60
<212> DNA
<213> Artificial Sequence

<220>
<223> artificial oligonucleotide probe

<400> 40
aactacgagc tgcctgatgg tcaagtcatc acaattggag ctgagagatt ccgttgccca 60

<210> 41
<211> 60
<212> DNA
<213> Artificial Sequence

<220>
<223> artificial oligonucleotide probe

<400> 41
aaacattgtt cttagtggcg gttcaaccat gttccctggt attgcagacc ggatgagcaa 60

<210> 42
<211> 60
<212> DNA
<213> Artificial Sequence

<220>
<223> artificial oligonucleotide probe

<400> 42
aacgagcttc gtgttgcccc ggaggagcat ccagttcttc tcactgaagc accactcaac 60

<210> 43
<211> 60
<212> DNA
<213> Artificial Sequence

<220>
<223> artificial oligonucleotide probe

<400> 43
aaacatccaa tgtcccagcc atgtatgttg ctatccaggc agttctttct ctatatgcca 60

<210> 44
<211> 60
<212> DNA
<213> Artificial Sequence

<220>
<223> artificial oligonucleotide probe

<400> 44
aaaaactacg agttacctga tggacaagtc atcaccattg gtgcagagag attcagatgc 60

<210> 45
<211> 60
<212> DNA
<213> Artificial Sequence

<220>
<223> artificial oligonucleotide probe

<400> 45
aaaattttga ctgagcgtgg ctattccttc accaccacag ctgagcgaga aattgtcagg 60

<210> 46
<211> 60
<212> DNA
<213> Artificial Sequence

<220>
<223> artificial oligonucleotide probe

<400> 46
aaacttccaa gacaagttct tctgttgaga agagctatga gttacctgat gggcaggtga 60

<210> 47
<211> 60
<212> DNA
<213> Artificial Sequence

<220>
<223> artificial oligonucleotide probe

<400> 47
aaacattgtc ctcagtggtg gttctaccat gttccctggc attgctgaca gaatgagcaa 60

<210> 48
<211> 60
<212> DNA
<213> Artificial Sequence

<220>
<223> artificial oligonucleotide probe

<400> 48
aaaagagcta tgagcttcct gatggccagg taatcaccat cggtgcagaa cggttcagat 60

<210> 49
<211> 60
<212> DNA
<213> Artificial Sequence

<220>
<223> artificial oligonucleotide probe

<400> 49
aaagacttgt atgggaatat tgtgctcagt ggcggttcca caatgtttcc agggattgca 60

<210> 50
<211> 60
<212> DNA
<213> Artificial Sequence

<220>
<223> artificial oligonucleotide probe

<400> 50
aaattacttc actggctccc agcagcatga agattaaggt ggttgcacct ccagaaagga 60

<210> 51
<211> 60
<212> DNA
<213> Artificial Sequence

<220>
<223> artificial oligonucleotide probe

<400> 51
cacaaatcat gtttgagacc ttcaatgttc cagccatgta tgtggccatc caggctgtgc 60

<210> 52
<211> 60
<212> DNA
<213> Artificial Sequence

<220>

<223> artificial oligonucleotide probe

<400> 52
aacgggaaat tgtaagggac atcaaggaga agctcgcata tgtggctctt gactatgaac 60

<210> 53
<211> 60
<212> DNA
<213> Artificial Sequence

<220>
<223> artificial oligonucleotide probe

<400> 53
aaaatgccaa gagcagctcc tctgtggaga agagctatga gctgcctgat gggcaggtga 60

<210> 54
<211> 60
<212> DNA
<213> Artificial Sequence

<220>
<223> artificial oligonucleotide probe

<400> 54
actgggatga tatggagaag atttggcatc acactttcta caatgagctt cgtgttgctc 60

<210> 55
<211> 60
<212> DNA
<213> Artificial Sequence

<220>
<223> artificial oligonucleotide probe

<400> 55
atgatatgga gaagatttgg catcacactt tctacaatga gcttcgtgtt gctcgtgaag 60

<210> 56
<211> 60
<212> DNA
<213> Artificial Sequence

<220>
<223> artificial oligonucleotide probe

<400> 56
acactttcta caatgagctt cgtgttgctc gtgaagagca cccagtcctc ctgactgagg 60

<210> 57
<211> 60
<212> DNA
<213> Artificial Sequence

<220>
<223> artificial oligonucleotide probe

<400> 57
ggagaagatc tggcatcaca ctttctacaa cgagctccgt gtagcccctg aggagcaccc 60

<210> 58
<211> 60
<212> DNA
<213> Artificial Sequence

<220>
<223> artificial oligonucleotide probe

<400> 58
aaacttgctt acgtcgctct agacttcgag caagagctgg agacagctaa gagcagttct 60

<210> 59
<211> 60
<212> DNA
<213> Artificial Sequence

<220>
<223> artificial oligonucleotide probe

<400> 59
aaacatcgtc ctcagtggtg gttcaaccat gttcccagga atcgctgacc gtatgagcaa 60

## Description

The present invention relates to the field of oligonucleotide hybridisation and detection methods.

Nucleic acid hybridisation is the backbone of many genomic detection and sequencing approaches as well as of expression analysis. Specificity of hybridization between probes and intended targets is always critical. Unspecific binding of not perfectly complementary target molecules to oligonucleotide probes is one of the main problems for biosensor and high-through-put microarray approaches. Molecules with few mismatches in a sample can lead to a false positive signal. One approach to this problem is optimizing the melting temperature of the probe oligonucleotides, in particular in the case of microarrays with many probes it is desired to select unitary melting temperatures of all probe molecules (e.g. as disclosed in WO 2002/070736), increasing stringency by selecting a hybridisation temperature near the melting temperature and including multiple steps of stringent washing. Further, alternative approaches aim at observing kinetics, microaggitation or selecting probes with a well defined secondary structure. Other attempts use competitive nucleotides with few mismatches which do not lead to a signal. The simplest approach is to select only short oligonucleotide probes which have inherently a high selectivity but suffer from a low sensitivity.

The goal of the present invention is to provide an improved method to selectively detect oligonucleotides in a sample and increase specificity. This method should also be suitable to increase specificity for long oligonucleotides which have inherently also a high sensitivity but usually suffer from low selectivity.

Therefore the present invention provides a method suitable for binding an oligonucleotide molecule of a sample to an oligonucleotide probe with increased specificity comprising the steps of

- providing a solid support with at least one immobilized oligonucleotide DNA probe comprising an oligonucleotide probe sequence,
- in any order, contacting said probes with a sample comprising oligonucleotide molecules potentially binding said probe sequence and contacting said probes with at least one competitive non-DNA oligonucleotide, wherein said competitive oligonucleotide comprises a sequence which is complementary to the oligonucleotide probe sequence with up to 15% mismatches; and/or as an alternative or in addition, in any order, contacting said probes with a sample comprising oligonucleotide molecules and contacting said oligonucleotide molecules with at least one competitive non-DNA oligonucleotide, wherein said competitive oligonucleotide comprises a sequence of the oligonucleotide probe sequence with up to 15% mismatches,
- incubating said contacted probes with the oligonucleotide molecules of the sample and competitive oligonucleotides at a temperature of between 15°C below the melting temperature of DNA duplex oligonucleotides of the probe sequence up to said melting temperature,

thereby increasing binding specificity of the oligonucleotide probe to an oligonucleotide molecule comprising a sequence complementary to said probe sequence.

Many biosensors or microarrays detect the hybridisation of a target oligonucleotide, also referred herein as oligonucleotide molecule of a sample to be detected, onto the probe. This probe is usually designed to detect one particular target sequence, i.e. a sequence complementary to the probe sequence. Apart from a portion encoding the specific probe sequence, the probe may contain further portions, in particular linker portions, or linker nucleic acids which aim at binding the probe to the solid support.

The present invention provides methods to selectively bind target oligonucleotides to probes in the knowledge of kinetic effects of the competitive replacement of different nucleic acids. This information has been obtained by monitoring the temporal development of binding events in comparison to the binding energy of the involved molecules. The effect of replacing false positive target molecules with one or more mismatches in general relates to the lower binding energy of these molecules.

By including the competitive non-DNA molecules it is now possible to increase specificity of biosensors or microarrays. Such biosensors or microarrays are examples of solid supports onto which the probes are immobilized. This immobilization can be covalent or by absorption. "Microarray" can be understood in the field as either a support onto which a multitude of oligonucleotide probes are immobilized or an arrangement of such probes. According to the invention, microarray shall read on both interpretations, e.g. a solid support with the immobilized probes, preferably more than 1 type of probe, in a microscopic arrangement.

The oligonucleotide probe comprises a sequence stretch complementary to a target molecule, the oligonucleotide probe sequence, and may also comprise other portions, in particular a linker portion. This linker portion reserved to covalently bind the probe to the solid support. The linker may or may not be an oligonucleotide stretch. It usually serves the purpose to distance the oligonucleotide probe sequence portion from the solid support to allow complete hybridisation over the complete probe sequence with target molecules.

By selecting a high probe length it is desired to increase sensitivity of the system by aquiring longer target oligonucleotide molecules in a sample to be analysed. However, this is usually the cause of problems and weaknesses in conventional microarrays. Usually, probes are designed to comprise a probe sequence which is complementary to a part of the DNA target sequence of equal length, with a 100% match. It was shown that different molecules in a sample which do not share complete identity with the sequence complementary to the probe sequence, i.e. comprising mismatches as compared to the desired DNA target, can bind, leading to unspecific binding. It was shown according to the invention that such non-complementary targets can be replaced from the probes by RNA or related nucleic acids, such as LNA (locked nucleic acids) or PNA (peptide nucleic acids) of similar sequence. This effect allows a substantial increase in the specificity of hybridisation reactions, since RNA or related nucleic acid molecules, in particular LNA, can bind strongly to the DNA probes.

It is not required that these competitive non-DNA oligonucleotides are 100% complementary to the oligonucleotide probe sequence (or to the DNA target sequence). It is sufficient that these competitive oligonucleotides comprise a sequence complementary to the oligonucleotide probe sequence with up to 15% mismatches. Such competitive oligonucleotides may bind to the probe without generating a signal. In an alternative to this embodiment it is also possible to use oligonucleotide probe sequences which do not bind the probes but bind the (intended) target oligonucleotide molecules of the sample and increase specificity by this binding event. According to this embodiment usually not the oligonucleotide bound to the probes are detected but those removed from the detection system. Also, the probes may be free of the target, bound by the competitive non-DNA oligonucleotide. In this case, the competitive oligonucleotide comprises a sequence identical with the oligonucleotide probe sequence, which is also allowed to have up to 15% mismatches.

"Mismatch" relates to a deviation of identity of the complementary base and can be a substitution, addition or deletion of the base sequence, in particular a substitution. In preferred embodiments either type of competitive oligonucleotide sequences preferably has up to 15%, up to 14%, up to 13%, up to 12%, up to 11%, in particular preferred up to 10%, up to 9%, up to 8%, up to 7%, up to 6%, up to 5%, up to 4%, up to 3%, up to 2%, or up to 1% mismatches. The number of these mismatches can be arithmetically rounded. There can be 0, 1, 2, 3, 4, 5, 6, 7, or 8 mismatches. The preferred range of mismatches is 1-4 mismatches. Even more preferred is 100% identity with the probe sequence or with a sequence complementary to the probe sequence.

In preferred embodiments the competitive oligonucleotide is a molecule of RNA, LNA, PNA or a mixture thereof or a mixture thereof with DNA. It is possible to have multiple nucleic acid backbone types in such a molecule. However, preferred are molecules with identical nucleic acid backbone, preferably only RNA. Preferably this competitive oligonucleotide is as least 50% RNA, preferrably 60% RNA, at least 70% RNA, at least 80% RNA, or at least 90% RNA. Particular preferred the competitive oligonucleotide is completely of RNA nucleotide types.

According to the inventive method it is also possible to include the step of detecting binding events of the oligonucleotide molecule and the oligonucleotide probe.

Such binding events can be detected by common methods used in the field, such as detecting labeled oligonucleotides on a solid support. It is possible to label target molecules which can then be detected upon binding to the oligonucleotide probes or, alternatively labelling competitive molecules which are displaced by the target molecules. Such competitive binding is usually dependent on the concentration of the target molecules in the sample which increasingly displace the labelled competitive molecules depending on the concentration. Preferably - but not exclusively - the label is a fluorescence label such as a Cy3 and/or Cy5 label which is detectable by conventional fluorescence readers.

The inventive method is in particular suitable to increase specificity of large oligonucleotide probes which usually suffer from cross-hybridisation. Therefore, preferably long oligonucleotide probe sequences are used. This oligonucleotide probe sequences may comprise at least 35, at least 36, at least 38, preferably at least 40, at least 42, at least 44, in particular preferred at least 45, at least 46, or at least 48 nucleotides. The probe sequences may be up to 80 nucleotides, up to 90 nucleotides, up to 100 nucleotides, up to 120 nucleotides, up to 200 nucleotides, up to 500 nucleotides, or even up to 1000 nucleotides, or up to 2000 nucleotides. A preferred length range is 40 to 80 nucleotides. Although, usually synthetic oligonucleotide probes are used. It is also possible to use complete cDNA probes.

In preferred embodiments the incubation step with the competitive non-DNA molecule is for at least 2, preferably at least 4, even more preferred at least 6, minutes. Further incubation step may be for at least 8, at least 10, at least 15, at least 20, at least 25, at least 30, at least 35, at least 40, at least 45, at least 50, or more minutes. Incubation serves the purpose of allowing the competitive reaction with the competitive non-DNA oligonucleotide to displace any cross-hybridising DNA targets in the sample. This allows a more selective binding of DNA targets complementary to the probe sequence. Suitable times for this competitive reaction can be selected by the skilled man in the art. The time usually depends on the order of the inventive method steps. For example, it is possible to first contact the probe with the sample oligonucleotides. This leads to a binding reaction of target sequences but also of cross-hybridizing unintentional target sequences to the probes. To sufficiently displace the cross hybridising sequences usually longer incubation times are preferred, in particular at least 50, at least 60, even more preferred at least 100 minutes. Sometimes incubation times of 1, 2, 3, 4, 5, or more hours can be necessary, depending on the probe sequence. On the other hand it is also possible to first contact the probe with the competitive oligonucleotides being complementary to the probe sequence or contact the oligonucleotide molecule of the sample first with the competitive oligonucleotide comprising the probe sequence. In this case the probe may bind such a competitive oligonucleotide which has in turn to be displaced by the target nucleotide. This reaction may be slower as compared to the displacement of DNA targets. Particular fast reactions are feasable by coincidentally contacting the probe with the sample and the competitive non-DNA oligonucleotides.

The inventive method is particularly suitable to analyse complex samples. Such complex samples may comprise a multitude of different oligonucleotide molecules which may cross-hybridise with the intended target/probe hybridisation reactions. In preferred embodiments the sample comprises at least 2, preferably at least 5, even more preferred at least 10, different oligonucleotide molecules.

It is also possible to increase specificity by using a multitude of different competitive non-DNA oligonucleotides. Preferably at least 2, at least 4, at least 5, at least 10, at least 12, at least 15, at least 20, at least 25, at least 50, at least 100, at least 200, at least 300 different non-DNA oligonucleotides are used which may be complementary to at least 1 or more of the probes on the solid support (with or without the mentioned mismatches). In preferred embodiments, at least one competitive non-DNA oligonucleotide is used for each probe on the support.

The incubation step is preferably performed at a temperature between 15°C below the melting temperature and the melting temperature itself (preferably still being below the melting temperature; e.g. 1°C lower than the melting temperature). Preferably the incubation temperature for the hybridisation reactions and displacement reactions is not lower than 12°C below the melting temperature, in particular not more than 10°C below the melting temperature, even more preferred not more than 8°C below the melting temperature, of a DNA:DNA duplexbinding to the probe sequence. The cross hybridising mismatch targets of asample usually have a lower melting temperature due to lower binding energy caused by the mismatches. The competitive oligonucleotides usually have a higher melting temperature and/or binding energy (e.g. at experimental conditions) due to stable DNA (probe): RNA (competitive oligo) duplex. According to the invention it was found that these non-DNA oligonucleotides can selectively displace DNA targets with this mismatches. This appears to be a kinetic phenomenon. The melting temperature, used as reference for the DNA:DNA duplex, cannot be given as an absolute value but depends on the GC content of the oligonucleotide probe but also the solution used during the incubation step. In preferred embodiments the melting temperature during the incubation step is modified by applying an organic solvent and/or a detergent.

The incubation step is usually performed in aqueous solution. The melting temperature can e.g. be lowered by detergents such as SSC or SDS or using organic solvents such as formamid. Usually the incubation step is performed in a physiological saline concentration (such as about 0.15 M) or higher (e.g. up to 0.9 M or more) using high degrees of organic solvents. The melting temperature can be decreased from the usually 96-98°C melting temperature to even room temperature.

In preferred embodiment the solid support is a chip, preferably a micoarray, or a biosensor. The solid support may comprise at least 2, preferably at least 4, even more preferred at least 8, at least 10, at least 15, at least 20, at least 25, at least 50, at least 100, at least 200, at least 300, or more probes comprising different probe sequences. The different probes can e.g. be arranged in isolated spots. Such spots can be read by conventional microarray reader to detect any hybridisation events thereon.

In another aspect of the present invention a kit is provided for performing any of the above inventive methods, in particular comprising a solid support with at least one immobilized oligonucleotide DNA probe comprising an oligonucleotide probe sequence and a container comprising a competitive oligonucleotide comprising a sequences being complementary to said probe sequence or identical to said probe sequence with up to 15% mismatches, wherein said competitive oligonucleotide is non-DNA, preferably RNA, LNA or PNA. The container can be any container used in the field to hold oligonucleotides, in particular vials. The competitive (non-DNA, e.g. RNA) oligonucleotides are usually provided in dried condition, preferably freeze dried condition.

The kit preferably further comprises detection means for detecting binding events between oligonucleotide molecules, preferably a label, in particular a fluorescence label, e.g. a Cy3 and/or Cy5 label. The kit may further comprise suitable buffers and solutions for the inventive methods.

In preferred embodiments of the kit the oligonucleotide probes, the solid support, the competitive oligonucleotides can be defined as mentioned above for the inventive method.

The present invention is further illustrated by the following figures and example, without being limited thereto.

Figures:

Fig. 1: Signal intensity profiles for RNA (a) and DNA (b) targets in a competitive displacement of DNA with RNA. The development of the fluorescence intensities over time illustrate the displacement of DNA targets with lower hybridisation energy (e.g. due to mismatches or weaker base pairing). In the graph for DNA targets (b) the downward curve (probe 14-652) illustrates a target-probe combination with low binding energy (mismatch). Similar results are shown for probe 14-175 highlighted in (c). Clearly shown is that not only does the RNA-hybridisation slow DNA hybridisation but also leads to replacement of DNA strands with low binding energy.

Fig. 2 shows the dependency of the competitive deplacement (y-axis) from the melting temperature of a DNA-DNA duplex (x-axis), in dependence of the number of mismatches. The melting temperature of all these experiments was at 42°C. In this case, the melting temperature was given for each oligonucleotide probe, which are 60mer oligonucleotides. With standard methods such a high resolution was not possible.

Fig. 3 shows a set of oligonucleotides and the number of mismatches to Actin sequences. Several probes were derived from potato (1), bell pepper (2), tobacco (5), oak (6), poplar (8), castanea (14) and pine (19) targets, among others. The indication on the left gives the specificity to each of these plants. In the first row the probe number is given with a first indicating the plant specificity (without mismatch) number, the second number indicates the starting position of the genomic origin of the 60mer probes.

Examples :

**Example 1: Extraction and purification of the Actin gene fragments:**

Using the degenerate primers ActinForward: ACT GGG ATG AYA TGG AGA AG (SEQ ID NO: 1) and Actin-Reverse AYC CTC CAA TCC AGA CAC TG (SEQ ID NO: 2), PCR-Amplification was performed on gDNA from the following species: potato, bell pepper, faba bean, tobacco, truffle oak, garden pea, black poplar, european raspberry, maize, soybean, sundew, european chestnut, devil's backbone, apple, scots pine, bread wheat, tree-of-heaven, oil-seed rape. PCR reaction of 50 μl volume, consisting of 10xPCR buffer (KTAQ, Kbioscience buffer B), 20 mM dNTP each, 5 U Taq polymerase (KTAQ, kbioscience), 4 μM primer each and 50 ng genomic DNA as template, were performed in a Dyade thermocycler (MJResearch Dyade). Amplification conditions were: 95°C for 5 min activation, then 34 cycles of: 30 s at 95°C, 30 s at annealing temperature55°C, 1 min at 72°C, followed by a final elongation step of 5 min at 72°C.

To ensure that only a single fragment of the actin gene family is used in the consecutive steps (McKinney and Meagher, 1998; Muller et al., 2007) the resulting PCR fragments were cloned using TA cloning kit (Clonetech) following providers instruction. Plasmid DNA was extracted from transformed E.coli using standard alkaline lysis protocol. After extraction cloned fragments were sequenced (AGOWA).

After sequencing the phylogenetic tree and standard microarray experiments were used to choose 6 out of the 18 genes for further analysis; two groups with very close homology i) potato, pepper, tobacco and ii) oak, poplar as well as iii) pinus with limited sequence homology to the other selected Actins.

**Example 2: Target preparation:**

RNA Targets/Competitive oligos:

For the RNA-targets in vitro transcription, purification and microarray hybridization were carried out as described earlier (Bodrossy et al., 2003) with minor modifications. DNA amplification was done by PCR (same protocol as above) with a reverse primer containing the T7 promoter site: 5'-TAATACGACTCACTATAG-ActinReverse-3' (SEQ ID NO: 3) as follows: 8 μl purified PCR product (50 ng/μl), 4 μl 5x T7 RNA polymerase buffer, 2 μl DTT (100 mM), 0.5 μl RNAsin (40 U/μl) (Promega), 1 μl each of ATP, CTP, GTP (10 mM), 0.5 μl UTP (10 mM), 1 μl T7 RNA polymerase (40 U/μl) (Gibco BRL) and 1 μl Cy3 or Cy5-UTP (5 mM) were added into a 1.5 ml microcentrifuge tube and incubated at 37°C for 4 h. RNA was purified immediately using the Quiagen RNeasy kit according to manufacturer's instructions. Purified RNA was eluted into 50 ml dH2O. PCR yield and incorporation (degree of labeling, DoL) measurement was done using a ND-100 Spectrophotometer (NanoDrop, Wilmington, DE).

ssDNA Targets:

For the preparation of ssDNA, a PCR was performed with an biotinylated forward primer and a regular reverse primer for the Actin gene. PCR condition were the same as above. PCR fragments were purified according to QIAquick PCR purification columns according to QIAquick spin Handbook. Forward strands were removed using Dynabeads® Streptavidin trial kit (Invitrogen DYNAL) according to the manufacturers protocol. Labeling with Cy3/Cy5 was performed using Createch ULS® arrayCGH labeling Kit according to manufacturers protocol.

PCR yield and incorporation (degree of labeling, DoL) measurement was done using a ND-100 Spectrophotometer (NanoDrop, Wilmington, DE).

Depending on the probe sequence, the target molecules act as analyte or as competitive target.

**Example 3: Probe design and preparation of the slides**

Probe design was carried out using the ARB phylogenetic software package (Ludwig et al 2004). Unmodified oligos synthet-isized by Sigma-Aldrich according to (Wang et al., 2003) were used. Figure 3 shows the number of mismatches for the most important probes, each having a probe sequence of 60 nucleotides (probe sequences from top to bottom: SEQ ID Nos: 4 to 59).

Every probe (dissolved in 50% DMSO) was spotted in three technical replicates to provide measurements of intra-microarray reproducibility. Probes for the 6 most important targets were spotted in four different concentrations (5.6, 16.7, 50.0 and 150 μM). With this setup evaluation of the influence of the spotting concentration on intensity and melting point measurements (spotshape) was possible. The 5.6 μM concentration was not used for analysis because the intensity was too weak in most of the cases. Spotting was performed on Genewave Amplislides® with Aldehyde coating using SMP3 stealth pins on a Omnigrid 100 platform. Slideprocessing was carried out according to (Bodrossy et al., 2003).

**Example 4: Hybridization**

Hybridization was carried out using the HybLive hybridization and scanning workstation (www.genewave.com). The hybridization buffer consisted of 6xSSC, 1x Dennhardt's reagent, 0.1 % SDS and 45% formamide. For hybridization we used prepared stock solutions to minimize the influence of mixing. The standard target concentration was set to 1 nM. To reduce the influence of secondary structure the hybridization solution was incubated at 95°C for 5 min and cooled on ice immediately before hybridization.

During the 12h hybridization time the temperature was set to 42°C and the HybLive®, agitation was on. The protocol of the hybridization at the Genewave Hyblive® was performed according to (Yann Marcy, 2008). After hybridization a washing step was performed for 5 min in 6x SSC and afterwards a high stringency wash for 5 min with 0.2xSSC, both at hybridization temperature. Before melting 15 exposures (1 each minute) at hybridization temperature have been done to ensure constant conditions and to see if there is any photobleaching. During measurements noimpact of photobleaching was seen. This might be due to the lower energy density of the LED based system compared to laser scanner. The melting analysis was done in hybridization solution from 42°C to 85°C with a ramp rate of 1 °C/min.

Normalization and data analysis was done with the HybLyze® software. All data presented here are the average of the three technical replicates if not stated different

**Example 5: Data Analysis:**

The kinetic curves were fitted with a three parameter fit. The fit function for the hybridization intensity was motivated by the formula for linked chemical reactions:

$$I_{SS} \xrightarrow{k_a} I_{DNA} \xrightarrow{k_b} I_{DNA-RNA}$$

where $k_a$ is the reaction constant of the DNA hybridization on the spot and $k_b$ is the rate constant of the displacement of DNA after competitive hybridization with RNA. $I_{DNA}$ was approximated with

$$I_{DNA} = A\frac{k_a}{k_b - k_a}\left(e^{-k_a t} - e^{-k_b t}\right)$$

The constants $k_a$ and $k_b$ allow estimation for the rate of hybridization and replacement of DNA. It is important to note that it is not an analytical model of the complex displacement reaction but a pure fitting algorithm to reduce the curves to the constants $k_a$ related to hybridization and kb to measure displacement and a constant A. Fig 1 gives an example of some fitted kinetic curves.

The goodness of fit (GOF) was monitored with $R^2$, were a threshold of $R^2$=0.99 was used. The fitting of the whole curve was chosen instead of using e.g. the derivation at the end of the hybridization because it was more accurate and less noise sensitive description of the hybridization kinetic. A positive value of $k_b$ indicates decreasing intensity thus displacement. Additionally the ratio

$$k_I = {I_{max}}/{I_{end}} - 1$$

was monitored, with the maximum intensity $I_{max}$ and the intensity at the end of the hybridization $I_{end}$. A value of $k_b > 0$ indicates displacement (Fig. 2).

The determination of the melting temperature was according to (Yann Marcy, 2008) with the Genewave made software Hyblizer™.

**Example 6: Results**

To evaluate the biological and physico-chemical relevance Actin sequence probes on a chip were investigated. Actin is one of the most conserved housekeeping genes in eukaryotes. Cross-species comparison of this gene provides a set of sequences with a broad range of homology levels. An extraction and amplification on 18 different plant species was done. Out of the 18 genes 6 were chosen for an evaluation of competitive hybridization 5 with very close homology and one (pinus) with less sequence homology with the other Actins.

According to the invention it was found that for probe/target duplexes with melting temperature close to the actual hybridization temperature DNA is replaced during hybridization by RNA. An example of the development of the fluorescence intensity is shown in fig. 1. According to this experiment slow replacement conditions have been selected to monitor the induced processes. However, faster reactions are also possible by changing the order of the contacting steps.

To investigate the competition of RNA and DNA targets equal concentrations of DNA and RNA targets of the same sequence and of sequences comprising mismatches were used. For control purposes a dye swap was done, the correlation (melting temperatures) is shown in fig 2 as a typical example of this behavior. In the example of fig. 2 the hybridization temperature was 42°C, increased stepwise, and the clear distinction between replaced targets at ~52°C is clearly visible. The dependence of the replacement reaction on the mismatches (MM) and melting temperatures of the targets is evident. This allows the use of competitive RNA in dependence on the melting temperature e.g. 10°C below the melting temperature, in order to increase stringency conditions of a hybridizing biosensor or microarray, e.g. the competitive displacement of DNA with RNA for non stringent binding is now a possibility to improve the specificity of microarrays. It is also possible to use it as an indicator for cross hybridization.

Reference List

Bodrossy et al. (2003) Environmental Microbiology 5: 566-582.
Bonnet et al. (1999) Proceedings of the National Academy of Sciences of the United States of America 96: 6171-6176.
Dai et al. (2002) Nucl. Acids Res. 30: e86.
McKinney et al. (1998) Genetics 149: 663-675.
Muller et al. (2007) BMC Genomics 8: 294.
Pozhitkov et al. (2008) Journal of Microbiological Methods 75: 92-102.
Toegl et al. (2003) Journal of Biomolecular Techniques 14: 197-204.
Wang et al. (2003) Genome Biology 4.
Wick et al. (2006) Nucl. Acids Res. 34: e26.
Yann et al. (2008) BioTechniques 44: 913-920.

## Claims

1. A method to bind oligonucleotide molecules of a sample to an oligonucleotide probe with increased specificity comprising the steps of

    - providing a solid support with at least one immobilized oligonucleotide DNA probe comprising an oligonucleotide probe sequence,
    - in any order, contacting said probes with a sample comprising oligonucleotide molecules potentially binding said probes and contacting said probes with at least one competitive non-DNA oligonucleotide, wherein said competitive oligonucleotide comprises a sequence which is complementary to the oligonucleotide probe sequence with up to 15% mismatches; and/or as an alternative or in addition, in any order, contacting said probes with a sample comprising oligonucleotide molecules and contacting said oligonucleotide molecules with at least one competitive non-DNA oligonucleotide, wherein said competitive oligonucleotide comprises a sequence of the oligonucleotide probe sequence with up to 15% mismatches,
    - incubating said contacted probes with the oligonucleotide molecules of the sample and competitive oligonucleotides at a temperature of between 15°C below the melting temperature of DNA duplex oligonucleotides of the probe sequence up to said melting temperature,

    thereby increasing binding specificity of the oligonucleotide probe to an oligonucleotide molecule comprising a sequence complementary to said probe sequence.

2. The method of claim 1, **characterized in that** the competitive oligonucleotide is a molecule of RNA, LNA, PNA or a mixture thereof or a mixture thereof with DNA.

3. The method of claim 1 or 2 further comprising the step of detecting binding events of the oligonucleotide molecule and the oligonucleotide probe.

4. The method of any one of claims 1 to 3, **characterized in that** the oligonucleotide probe sequence comprises at least 35, preferably at least 40, even more preferred at least 45, nucleotides.

5. The method of any one of claims 1 to 4, **characterized in that** the oligonucleotide probe comprises a linker portion and said linker portion is covalently bound to said solid support.

6. The method of any one of claims 1 to 5, **characterized in that** the incubation step is for at least 2, preferably at least 4, even more preferred at least 6, minutes.

7. The method of any one of claims 1 to 6, **characterized in that** the probe is first contacted with the sample oligonucleotides.

**8.** The method of any one of claims 1 to 6, **characterized in that** the probe is first contacted with the oligonucleotide molecule of the sample potentially being complementary to the probe sequence, or the oligonucleotide molecule of the sample is first contacted with the competitive oligonucleotide comprising the probe sequence.

**9.** The method of claim 7, **characterized in that** the incubation step is for at least 50, preferably at least 60, even more preferred at least 100, minutes.

**10.** The method of any one of claims 1 to 9, **characterized in that** the sample comprises at least 2, preferably at least 5, even more preferred at least 10, different oligonucleotide molecules.

**11.** The method of any one of claims 1 to 10, **characterized in that** the melting temperature during the incubation step is modified by applying an organic solvent and/or a detergent.

**12.** The method of any one of claims 1 to 11, **characterized in that** the solid support is a chip, preferably a micoarray, or a biosensor.

**13.** The method of any one of claims 1 to 12, **characterized in that** the solid support comprises at least 2, preferably at least 4, even more preferred at least 8, probes comprising different probe sequences.

**14.** A kit for performing a method according to any one of claims 1 to 13, comprising a solid support with at least one immobilized oligonucleotide DNA probe comprising an oligonucleotide probe sequence and a container comprising a competitive oligonucleotide comprising a sequences being complementary to said probe sequence or identical to said probe sequence with up to 15% mismatches, wherein said competitive oligonucleotide is non-DNA, preferably RNA, LNA or PNA.

**15.** The kit of claim 14 further comprising detection means for detecting binding events between oligonucleotide molecules, preferably a label, in particular a fluorescence label.

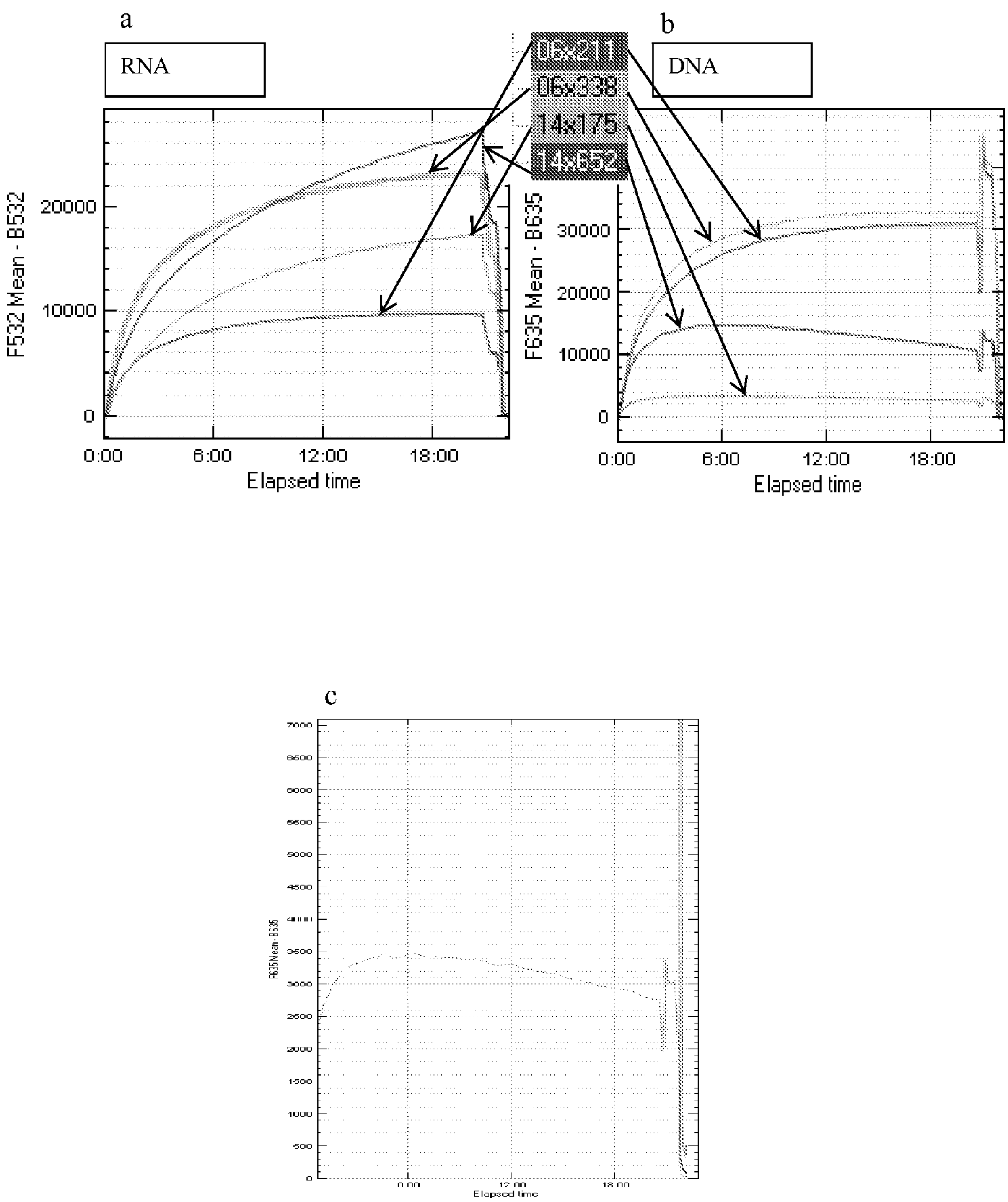
a
RNA
b
DNA
06x211
06x338
14x175
14x652
F532 Mean - B532
20000
10000
0
0:00
6:00
12:00
18:00
Elapsed time
F635 Mean - B635
30000
20000
10000
0
0:00
6:00
12:00
18:00
Elapsed time
c
7000
6500
6000
5500
5000
4500
3500
3000
2500
2000
1500
1000
500
0
6:00
12:00
18:00
Elapsed time

Fig. 1

Fig. 2

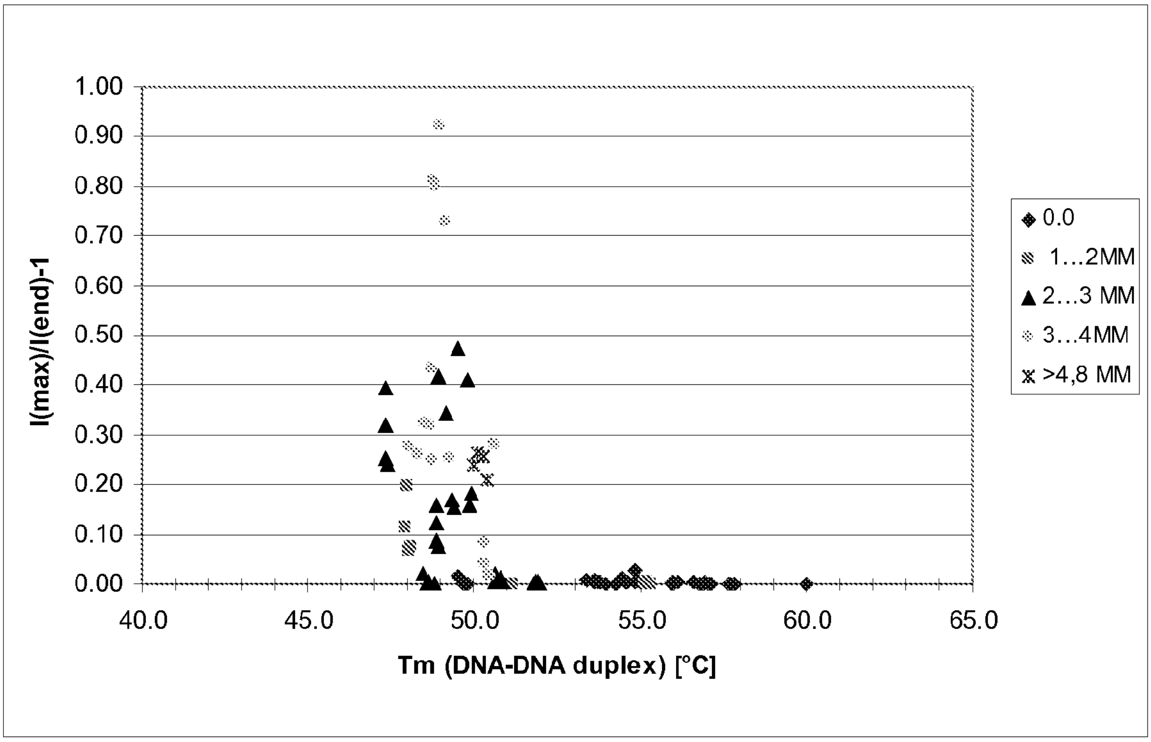
1.00
0.90
0.80
0.70
0.60
0.50
0.40
0.30
0.20
0.10
0.00
I(max)/I(end)-1
40.0
45.0
50.0
55.0
60.0
65.0
Tm (DNA-DNA duplex) [°C]
0.0
1…2MM
2…3 MM
3…4MM
>4,8 MM

| 22_Rape | 21_Gotebaum | 20_Wheat | 19_Pinus | 18_Malus | 16_Kalanchoe | 14_Castanea | 13_Drosera | 12_Soja | 11_Maize | 10_Rubus | 8_poplar | 7_Pisum | 6_oak | 5_Tabacco | 3_Vicia | 2_Pepper | 1_Potato | length | SEQTENCE | PROBE |
|---|---|---|---|---|---|---|---|---|---|---|---|---|---|---|---|---|---|---|---|---|
| 8 | 9 | 8 | 13 | 8 | 6 | 8 | 12 | 9 | 9 | 9 | 8 | 12 | 8 | 3 | 9 | 3 | 0 | 60 | ACTTTCTACAATGAGCTTCGTGTTGCCCCCGAGGAGCACCCTGTTCTGCTCACTGAAGCA | 01-032 |
| 15 | 14 | 12 | 15 | 19 | 14 | 14 | 18 | 11 | 13 | 16 | 14 | 7 | 15 | 5 | 15 | 4 | 0 | 60 | TTTGCCACATGCCATTCTTCGTTTGGATCTTGCTGGCCGTGATTTAACTGATAACCTGAT | 01-277 |
| 9 | 8 | 6 | 11 | 8 | 3 | 7 | 7 | 9 | 13 | 8 | 8 | 8 | 7 | 3 | 10 | 4 | 0 | 60 | AGGTATCCACGAGACTACCTACAACTCTATTATGAAGTGTGATGTTGATATCAGGAAGGA | 01-583 |
| 7 | 7 | 8 | 8 | 9 | 8 | 8 | 8 | 8 | 12 | 7 | 7 | 8 | 9 | 3 | 11 | 0 | 2 | 60 | AAATGACCCAGATTATGTTTGAGACCTTCAATGTTCCGGCCATGTATGTTGCTATTCAGG | 02-120 |
| 14 | 10 | 13 | 7 | 9 | 9 | 10 | 13 | 12 | 12 | 5 | 10 | 8 | 10 | 5 | 8 | 0 | 4 | 60 | AAATTACTGCTTTGGCTCCCAGCAGCATGAAGATTAAGGTTGTTGCACCACCAGAGAGAA | 02-714 |
| 15 | 15 | 14 | 23 | 13 | 15 | 12 | 16 | 15 | 10 | 11 | 15 | 18 | 12 | 16 | 0 | 13 | 14 | 60 | AAAAGCTGTCATACATTGCCCTTGACTATGAGCAAGAGCTGGAGACAGCGAGGACCAGCT | 03-408 |
| 14 | 17 | 14 | 14 | 12 | 14 | 14 | 16 | 16 | 18 | 15 | 17 | 16 | 14 | 14 | 0 | 14 | 14 | 60 | AAAGACTTGTATGGTAACATTGTCCTTTCAGGAGGAACAACCATGTTCCCAGGAATTGCT | 03-638 |
| 12 | 11 | 11 | 11 | 9 | 10 | 12 | 13 | 14 | 13 | 8 | 11 | 13 | 12 | 10 | 0 | 7 | 10 | 60 | AAAGAAATTTCTGCTTTGGCCCCAAGCAGCATGAAGATCAAGGTCGTTGCACCACCTGAG | 03-710 |
| 12 | 9 | 11 | 12 | 8 | 10 | 12 | 11 | 9 | 10 | 11 | 9 | 9 | 13 | 0 | 16 | 4 | 2 | 60 | AAAGGCCAACAGAGAGAAAATGACCCAGATTATGTTTGAGACATTCAACGTTCCGGCTAT | 05-103 |
| 8 | 6 | 9 | 14 | 10 | 7 | 5 | 7 | 10 | 12 | 7 | 6 | 10 | 5 | 0 | 14 | 3 | 3 | 60 | AAATTGTCCGTGACATGAAGGAGAAGCTTGCTTATGTGGCTCTTGACTACGAGCAGGAGC | 05-387 |
| 12 | 7 | 9 | 16 | 10 | 10 | 6 | 11 | 13 | 12 | 10 | 7 | 12 | 6 | 0 | 18 | 4 | 5 | 60 | ACATGAAGGAGAAGCTTGCTTATGTGGCTCTTGACTACGAGCAGGAGCTTGACACTGCCA | 05-399 |
| 12 | 10 | 10 | 10 | 7 | 5 | 8 | 9 | 8 | 14 | 7 | 10 | 8 | 9 | 0 | 12 | 6 | 4 | 60 | AAGTCCTCTTCCAGCCATCCATGATCGGAATGGAAGCTGCAGGTATCCATGAGACTACCT | 05-543 |
| 11 | 6 | 11 | 13 | 11 | 7 | 5 | 10 | 8 | 7 | 7 | 6 | 11 | 0 | 7 | 10 | 6 | 6 | 60 | AACTGCTATTGTGCTGGACTCTGGTGACGGTGTGAGTCGCACTGTGCCTATCTACGAAGG | 06-211 |
| 5 | 6 | 9 | 12 | 15 | 7 | 3 | 8 | 6 | 13 | 8 | 6 | 6 | 0 | 5 | 17 | 5 | 5 | 60 | AAGATTCTCACCGAGAGAGGGTACATGTTCACAACCACTGCTGAGCGGGAAATTGTCCGT | 06-338 |
| 9 | 6 | 5 | 15 | 11 | 8 | 2 | 10 | 9 | 6 | 8 | 6 | 8 | 0 | 10 | 12 | 9 | 8 | 60 | AACCATGTTCCCTGGTATTGCTGACCGGATGAGCAAGGAGATCACTGCACTTGCCCCAAG | 06-676 |
| 10 | 5 | 6 | 12 | 12 | 7 | 10 | 11 | 6 | 11 | 6 | 5 | 0 | 11 | 7 | 10 | 7 | 7 | 60 | AACAGGTATTGTCTTGGATTCTGGTGATGGTGTGAGTCATACCGTGCCAATCTATGAGGG | 07-211 |
| 12 | 10 | 12 | 17 | 19 | 9 | 7 | 12 | 6 | 23 | 12 | 10 | 0 | 9 | 9 | 15 | 9 | 9 | 60 | AACTGAATCTTTGATGAAGATCCTCACTGAGAGAGGGTATATGTTCACCACCTCGGCTGA | 07-322 |
| 14 | 12 | 10 | 13 | 11 | 14 | 11 | 11 | 12 | 13 | 13 | 12 | 0 | 11 | 11 | 17 | 10 | 11 | 60 | AAAGAGAAGCTTGCCTATGTTGCTGTGGATTATGAACAAGAGCTTGAAACTGCAAAGAGC | 07-404 |
| 5 | 1 | 5 | 10 | 4 | 6 | 2 | 5 | 5 | 5 | 4 | 0 | 6 | 3 | 6 | 4 | 4 | 4 | 60 | ACTGGGATGACATGGAGAAGATTTGGCATCACACTTTCTACAATGAGCTTCGTGTTGCTC | 08-001 |
| 6 | 2 | 6 | 11 | 5 | 8 | 2 | 7 | 7 | 6 | 5 | 0 | 7 | 3 | 8 | 5 | 5 | 6 | 60 | ATGACATGGAGAAGATTTGGCATCACACTTTCTACAATGAGCTTCGTGTTGCTCCTGAAG | 08-006 |
| 10 | 1 | 6 | 15 | 11 | 11 | 4 | 11 | 9 | 9 | 7 | 0 | 9 | 4 | 11 | 9 | 7 | 9 | 60 | ACACTTTCTACAATGAGCTTCGTGTTGCTCCTGAAGAGCACCCAGTCCTCCTGACTGAGG | 08-030 |
| 9 | 0 | 7 | 10 | 10 | 8 | 6 | 6 | 7 | 8 | 6 | 0 | 6 | 7 | 7 | 4 | 6 | 6 | 60 | CTGAGGCTCCTCTCAACCCTAAGGCTAACAGAGAGAAGATGACTCAAATTATGTTTGAGA | 08+21-0 |
| 11 | 0 | 10 | 10 | 9 | 11 | 5 | 6 | 10 | 11 | 5 | 0 | 9 | 5 | 9 | 16 | 12 | 10 | 60 | CAGAAGTCCCCTTCCAGCCTTCTCTCATTGGAATGGAAGCTGCTGGCATCCACGAGACTA | 08+21-5 |
| 10 | 11 | 9 | 18 | 15 | 13 | 12 | 14 | 12 | 10 | 0 | 11 | 13 | 12 | 10 | 11 | 11 | 12 | 60 | AAAAACTTGCATACATGGCTCTTGACTACGAACAAGAGCTGGAGACTGCAAAGAGCAGCT | 10-408 |
| 9 | 5 | 12 | 11 | 13 | 9 | 7 | 12 | 13 | 15 | 0 | 5 | 12 | 8 | 12 | 13 | 10 | 11 | 60 | AAAAGAACTACGAGCTTCCTGATGGTCAGGTCATTACCATTGGAGCTGAGAGATTCAGAT | 10-477 |
| 12 | 9 | 9 | 10 | 11 | 5 | 9 | 11 | 12 | 10 | 0 | 9 | 9 | 9 | 8 | 8 | 5 | 7 | 60 | AAATTACTGCTCTTGCTCCCAGCAGCATGAAGATCAAGGTTGTGGCACCACCGGAGAGAA | 10-714 |
| 11 | 11 | 11 | 12 | 12 | 10 | 12 | 11 | 13 | 0 | 14 | 11 | 13 | 11 | 9 | 14 | 11 | 10 | 60 | AAACAGGGAGAAGATGACCCAGATTATGTTCGAGACATTCAACTGCCCAGCAATGTATGT | 11-109 |
| 15 | 10 | 13 | 15 | 11 | 14 | 10 | 17 | 12 | 0 | 10 | 10 | 13 | 10 | 13 | 10 | 9 | 10 | 60 | AAAAACTTGCCTACGTTGCCCTTGATTATGAACAGGAGCTGGAGACTGCCAGGACCAGCT | 11-408 |
| 14 | 15 | 9 | 15 | 16 | 14 | 14 | 14 | 13 | 0 | 13 | 15 | 13 | 13 | 14 | 18 | 14 | 11 | 60 | AAAGGTTTAGGTGCCCTGAGGTTCTATTCCAGCCATCCTTCATTGGCATGGAATCTGCTG | 11-525 |
| 10 | 8 | 8 | 9 | 10 | 8 | 5 | 7 | 0 | 8 | 5 | 7 | 4 | 7 | 9 | 7 | 10 | 7 | 60 | AAGATCTGGCATCACACATTTTATAATGAACTTCGTGTTGCTCCCGAGGAGCACCCAGTG | 12-017 |
| 13 | 11 | 15 | 17 | 14 | 15 | 9 | 15 | 0 | 12 | 13 | 11 | 11 | 9 | 15 | 15 | 15 | 16 | 60 | ACTTGCATATGTTGCCCTAGATTATGAGCAAGAACTCGAGACTGCAAAAAGCAGTTCATC | 12-412 |
| 12 | 11 | 13 | 14 | 14 | 15 | 12 | 16 | 0 | 16 | 11 | 11 | 8 | 12 | 14 | 13 | 16 | 18 | 60 | AACTCGAGACTGCAAAAAGCAGTTCATCAGTTGAGAAAAGCTATGAGCTTCCTGATGGAC | 12-444 |
| 3 | 8 | 7 | 7 | 13 | 8 | 5 | 0 | 6 | 11 | 7 | 8 | 6 | 6 | 11 | 9 | 9 | 11 | 60 | ACAGGGAAAAGATGACTCAGATCATGTTTGAGACGTTCAATGTCCCTGCCATGTATGTCG | 13-111 |
| 13 | 13 | 14 | 16 | 15 | 14 | 13 | 0 | 14 | 17 | 14 | 13 | 10 | 12 | 11 | 17 | 10 | 10 | 60 | AAACCGCTAAGAGTAGCTCTTCCATTGAGAAGAACTATGAACTTCCGGATGGGCAAGTTA | 13-450 |
| 14 | 7 | 8 | 13 | 10 | 8 | 12 | 0 | 9 | 8 | 10 | 7 | 10 | 11 | 8 | 17 | 7 | 7 | 60 | AAAGGACTTGTACGGCAACATTGTGCTCAGTGGTGGCTCTACTATGTTCCCTGGTATAGC | 13-637 |
| 8 | 8 | 14 | 8 | 8 | 8 | 0 | 9 | 9 | 7 | 5 | 8 | 11 | 3 | 11 | 12 | 9 | 10 | 60 | CCAGGCCGTTCTCTCTCTGTATGCCAGTGGTCGTACTACTGGTATTGTGCTTGACTCTGG | 14-175 |
| 5 | 4 | 9 | 17 | 12 | 8 | 0 | 13 | 7 | 13 | 6 | 4 | 6 | 3 | 9 | 14 | 8 | 9 | 60 | AACTACGAGCTGCCTGATGGTCAAGTCATCACAATTGGAGCTGAGAGATTCCGTTGCCCA | 14-482 |
| 9 | 8 | 9 | 12 | 8 | 10 | 0 | 12 | 6 | 12 | 4 | 8 | 6 | 2 | 9 | 14 | 8 | 8 | 60 | AAACATTGTTCTTAGTGGCGGTTCAACCATGTTCCCTGGTATTGCAGACCGGATGAGCAA | 14-652 |
| 10 | 12 | 11 | 14 | 9 | 0 | 10 | 11 | 9 | 15 | 6 | 11 | 11 | 10 | 4 | 12 | 7 | 6 | 60 | AACGAGCTTCCTGTTGCCCCGGAGGAGCATCCAGTTCTTCTCACTGAAGCACCACTCAAC | 16-041 |
| 8 | 11 | 10 | 9 | 12 | 0 | 9 | 11 | 13 | 12 | 8 | 11 | 13 | 9 | 13 | 14 | 11 | 14 | 60 | AAACATCCAATGTCCCAGCCATGTATGTTGCTATCCAGGCAGTTCTTTCTCTATATGCCA | 16-141 |
| 8 | 10 | 12 | 12 | 11 | 0 | 9 | 12 | 9 | 14 | 8 | 10 | 11 | 8 | 10 | 13 | 9 | 10 | 60 | AAAAACTACGAGTTACCTGATGGACAAGTCATCACCATTGGTGCAGAGAGATTCAGATGC | 16-479 |
| 14 | 12 | 12 | 13 | 0 | 19 | 14 | 13 | 14 | 12 | 16 | 12 | 14 | 15 | 15 | 8 | 15 | 15 | 60 | AAAATTTTGACTGAGCGTGGCTATTCCTTCACCACCACAGCTGAGCGAGAAATTGTCAGG | 18-338 |
| 14 | 12 | 9 | 15 | 0 | 14 | 15 | 15 | 14 | 13 | 14 | 12 | 13 | 14 | 14 | 13 | 15 | 15 | 60 | AAACTTCCAAGACAAGTTCTTCTGTTGAGAAGAGCTATGAGTTACCTGATGGGCAGGTGA | 18-450 |
| 7 | 6 | 9 | 13 | 0 | 10 | 8 | 8 | 11 | 10 | 6 | 6 | 9 | 6 | 9 | 12 | 8 | 8 | 60 | AAACATTGTCCTCAGTGGTGGTTCTACCATGTTCCCTGGCATTGCTGACAGAATGAGCAA | 18-652 |
| 13 | 12 | 10 | 0 | 11 | 13 | 16 | 12 | 13 | 9 | 11 | 12 | 13 | 15 | 15 | 12 | 15 | 14 | 60 | AAAAGAGCTATGAGCTTCCTGATGGCCAGGTAATCACCATCGGTGCAGAACGGTTCAGAT | 19-477 |
| 14 | 12 | 12 | 0 | 10 | 10 | 13 | 12 | 14 | 13 | 12 | 12 | 13 | 14 | 13 | 14 | 13 | 13 | 60 | AAAGACTTGTATGGGAATATTGTGCTCAGTGGCGGTTCCACAATGTTTCCAGGGATTGCA | 19-638 |
| 12 | 12 | 12 | 0 | 14 | 9 | 11 | 11 | 15 | 10 | 10 | 12 | 10 | 11 | 12 | 12 | 7 | 10 | 60 | AAATTACTTCACTGGCTCCCAGCAGCATGAAGATTAAGGTGGTTGCACCTCCAGAAAGGA | 19-714 |
| 11 | 6 | 0 | 10 | 8 | 10 | 8 | 9 | 4 | 12 | 8 | 6 | 4 | 8 | 11 | 11 | 8 | 9 | 60 | CACAAATCATGTTTGAGACCTTCAATGTTCCAGCCATGTATGTGGCCATCCAGGCTGTGC | 20-126 |
| 15 | 14 | 0 | 17 | 12 | 7 | 8 | 8 | 14 | 11 | 10 | 14 | 12 | 9 | 8 | 17 | 8 | 8 | 60 | AACGGGAAATTGTAAGGGACATCAAGGAGAAGCTCGCATATGTGGCTCTTGACTATGAAC | 20-381 |
| 15 | 8 | 0 | 14 | 9 | 14 | 9 | 14 | 13 | 12 | 10 | 8 | 11 | 8 | 10 | 15 | 12 | 12 | 60 | AAAATGCCAAGAGCAGCTCCTCTGTGGAGAAGAGCTATGAGCTGCCTGATGGGCAGGTGA | 20-450 |
| 6 | 0 | 4 | 9 | 5 | 5 | 3 | 4 | 6 | 6 | 3 | 1 | 7 | 2 | 5 | 5 | 3 | 3 | 60 | ACTGGGATGATATGGAGAAGATTTGGCATCACACTTTCTACAATGAGCTTCGTGTTGCTC | 21-001 |
| 8 | 0 | 6 | 11 | 7 | 8 | 4 | 7 | 9 | 8 | 5 | 2 | 9 | 3 | 8 | 7 | 5 | 6 | 60 | ATGATATGGAGAAGATTTGGCATCACACTTTCTACAATGAGCTTCGTGTTGCTCGTGAAG | 21-006 |
| 11 | 0 | 7 | 16 | 12 | 12 | 5 | 12 | 10 | 10 | 8 | 1 | 10 | 5 | 12 | 10 | 8 | 10 | 60 | ACACTTTCTACAATGAGCTTCGTGTTGCTCGTGAAGAGCACCCAGTCCTCCTGACTGAGG | 21-030 |
| 0 | 7 | 2 | 8 | 10 | 6 | 6 | 11 | 9 | 5 | 4 | 5 | 8 | 8 | 6 | 9 | 7 | 5 | 60 | GGAGAAGATCTGGCATCACACTTTCTACAACGAGCTCCGTGTAGCCCCTGAGGAGCACCC | 22-013 |
| 0 | 11 | 14 | 18 | 17 | 15 | 14 | 15 | 12 | 14 | 9 | 11 | 14 | 14 | 11 | 14 | 14 | 15 | 60 | AAACTTGCTTACGTCGCTCTAGACTTCGAGCAAGAGCTGGAGACAGCTAAGAGCAGTTCT | 22-410 |
| 0 | 9 | 8 | 14 | 7 | 5 | 9 | 13 | 11 | 12 | 6 | 9 | 8 | 7 | 8 | 13 | 8 | 8 | 60 | AAACATCGTCCTCAGTGGTGGTTCAACCATGTTCCCAGGAATCGCTGACCGTATGAGCAA | 22-052 |

Fig. 3

Europäisches Patentamt
European Patent Office
Office européen des brevets

# EUROPEAN SEARCH REPORT

Application Number
EP 09 16 4052

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 98/24933 A (BOSTON PROBES INC [US]; DAKO AS [DK]; COULL JAMES M [US]; HYLDIG NIELS) 11 June 1998 (1998-06-11)<br>* page 21 - page 24; claims 13,24,25,34 *<br>* the whole document * | 1-6, 8-11, 13-15<br>7,12 | INV.<br>C12Q1/68 |
| Y | FIANDACA M J ET AL: "PNA BLOCKER PROBES ENHANCE SPECIFICITY IN PROBE ASSAYS" PEPTIDE NUCLEIC ACIDS : PROTOCOLS AND APPLICATIONS, WYMONDHAM : HORIZON SCIENTIFIC PRESS, GB, 1 January 1999 (1999-01-01), pages 129-141, XP001160705<br>ISBN: 978-1-898486-16-9<br>* the whole document * | 1-15 | |
| Y | US 5 434 047 A (ARNOLD JR LYLE J [US]) 18 July 1995 (1995-07-18)<br>* the whole document * | 1-15 | |
| A | EP 1 435 394 A (FUJI PHOTO FILM CO LTD [JP]) 7 July 2004 (2004-07-07)<br>* the whole document * | 1-15 | TECHNICAL FIELDS SEARCHED (IPC)<br>C12Q |
| A | WO 02/18616 A (HITACHI CHEMICAL CO LTD [JP]; HITACHI CHEMICAL RES CT INC [US]; KE SON) 7 March 2002 (2002-03-07)<br>* the whole document * | 1-15 | |

The present search report has been drawn up for all claims

| Place of search | Date of completion of the search | Examiner |
|---|---|---|
| Munich | 20 August 2009 | Schmitt, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 09 16 4052

This annex lists the patent family members relating to the patent documents cited in the above-mentioned European search report.
The members are as contained in the European Patent Office EDP file on
The European Patent Office is in no way liable for these particulars which are merely given for the purpose of information.

20-08-2009

| Patent document cited in search report | Publication date | Patent family member(s) | Publication date |
|---|---|---|---|
| WO 9824933 A | 11-06-1998 | AU 7625998 A | 29-06-1998 |
| | | DE 69706315 D1 | 27-09-2001 |
| | | DE 69706315 T2 | 08-05-2002 |
| | | DK 946750 T3 | 17-12-2001 |
| | | EP 0946750 A1 | 06-10-1999 |
| | | US 2003124521 A1 | 03-07-2003 |
| US 5434047 A | 18-07-1995 | NONE | |
| EP 1435394 A | 07-07-2004 | JP 4185763 B2 | 26-11-2008 |
| | | JP 2004187603 A | 08-07-2004 |
| | | US 2005032080 A1 | 10-02-2005 |
| WO 0218616 A | 07-03-2002 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2002070736 A


### Non-patent literature cited in the description


- **Bodrossy et al.** *Environmental Microbiology,* 2003, vol. 5, 566-582
- **Bonnet et al.** *Proceedings of the National Academy of Sciences of the United States of America,* 1999, vol. 96, 6171-6176
- **Dai et al.** *Nucl. Acids Res.,* 2002, vol. 30, e86
- **McKinney et al.** *Genetics,* 1998, vol. 149, 663-675
- **Muller et al.** *BMC Genomics,* 2007, vol. 8, 294
- **Pozhitkov et al.** *Journal of Microbiological Methods,* 2008, vol. 75, 92-102
- **Toegl et al.** *Journal of Biomolecular Techniques,* 2003, vol. 14, 197-204
- **Wang et al.** *Genome Biology,* 2003, vol. 4
- **Wick et al.** *Nucl. Acids Res.,* 2006, vol. 34, e26
- **Yann et al.** *BioTechniques,* 2008, vol. 44, 913-920